(19) Europäisches Patentamt
European Patent Office
Office européen des brevets

(11) Publication number: 0 390 115 A1

# (12) EUROPEAN PATENT APPLICATION

(21) Application number: 90105923.8

(51) Int. Cl.⁵: C12P 21/08, C12N 5/12

(22) Date of filing: 28.03.90

The applicant has filed a statement in accordance with Rule 28 (4) EPC (issue of a sample only to an expert). Accession number(s) of the deposit(s): FERM BP-2330.

A request for correction of claim 6 has been filed pursuant to Rule 88 EPC. A decision on the request will be taken during the proceedings before the Examining Division (Guidelines for Examination in the EPO, A-V, 2.2).

(30) Priority: 29.03.89 JP 79890/89

(43) Date of publication of application: 03.10.90 Bulletin 90/40

(84) Designated Contracting States: AT BE CH DE DK ES FR GB GR IT LI LU NL SE

(71) Applicant: Yamashina, Ikuo
2 Shimogamohigashimorigamae-cho
Sakyo-ku
Kyoto-shi Kyoto(JP)

(72) Inventor: Yamashina, Ikuo
2 Shimogamohigashimorigamae-cho
Sakyo-ku
Kyoto-shi Kyoto(JP)

(74) Representative: Vossius & Partner
Siebertstrasse 4 P.O. Box 86 07 67
D-8000 München 86(DE)

## (54) Monoclonal antibody NNY128.

(57) A monoclonal antibody recognizing the following glycopeptide structure including N-acetylgalactosamine (GalNAc) of a mucin type glycopeptide is provided.

$$\begin{array}{ccccc} \text{Ser} & — & \text{Thr} & — & \text{Thr} \\ \uparrow & & \uparrow & & \uparrow \\ \text{R} & & \text{R} & & \text{R} \end{array}$$

wherein R is GalNAc.

EP 0 390 115 A1

## Monoclonal Antibody NNY128

This invention relates to a monoclonal antibody recognizing glycopeptide structures including N-acetylgalactosamine (GalNAc) of a mucin type glycopeptide, some of which are present on the surface of cancer cells and some are secreted, and to a hybridoma producing the monoclonal antibody.

On the surface of cells there are carbohydrate complexes such as glycoproteins, glycolipids, and proteoglycans. When normal cells are transformed into cancer cells, the sugar chains of these materials undergo changes.

The changes of sugar chains have been investigated mainly by isolating and chemically analyzing the sugar chains. However, ever since the technique for producing monoclonal antibodies was established by Köhler and Milstein in 1975, many attempts have been made to produce a monoclonal antibody which specifically reacts with varied sugar chains on the surface of cancer cells. Since it was known that most of the monoclonal antibodies recognizing cancer-associated antigens recognize sugar chains, the importance of monoclonal antibodies recognizing sugar chain antigens has been emphasized. Some of the sugar chain structures recognized by these antibodies have been determined. Such a monoclonal antibody surely plays an extremely important role in cancer investigation as well as in cancer therapy, such as diagnosis, inspection and treatment of cancer. So far the structure recognized by the antibody of the present invention, which has been called Tn antigen heretofore, has been unknown. The Tn antigen is very frequently detected in the cancer of epitheliocyte such as cancer of the alimentary canal, breast carcinoma and lung cancer (G. F. Springer et al., Carbohydr. Res. 178 , 271 (1988)). Furthermore, the Tn antigen is sometimes, though rarely, present on the surface of erythrocytes, which is called the Tn syndrome. As the Tn antigen disappears when treated with α-N-acetylgalactosaminidase, it seemed to have the structure GalNAcα → Ser/Thr (G. F. Springer et al., Carbohydr. Res. 178, 271 (1988)). However, this was no more than the partial structure of the epitope, and therefore an excellent antibody suitable to determine the complete structure has long been desired.

If the epitope of the Tn antigen is determined using anti-bodies, then the structure of a main cancer antigen can be determined.

Conventional monoclonal antibodies have been prepared by the following methods: (1) selecting a suitable antibody which binds to cancer cells which are used as an immunogen but not to the corresponding normal cells, or (2) using a mixture of glycolipids extracted from cancer cells with an appropriate adjuvant as an immunogen. It was, however, almost impossible to selectively obtain an antibody recognizing sugar chains of glycoproteins, some of which are present on the surface of cancer cells while others are secreted, because most antibodies for glycoprotein recognize its peptide portions.

A method for measuring a titer of an antibody for sugar chains of glycoproteins using immobilized glycopeptides is disclosed in JP-A-61-212568. By using this method, a monoclonal antibody recognizing sugar chains can efficiently be selected from many monoclonal antibodies produced by using cancer cells as an immunogen. According to the present invention, a monoclonal antibody recognizing only sugar chains of mucin type glycoproteins and being different from the already prepared MLS102 JP-A- 63-33398) in terms of an affinity for sialic acid was selected from said monoclonal antibodies and it was then shown that the antibody recognizes the Tn antigen. This invention relates to a monoclonal antibody recognizing glycopeptide structures including N-acetylgalactosamine (GalNAc) of a mucin type glycopeptide, some of which are present on the surface of cancer cells while others are secreted and to the production of the monoclonal antibody. Specifically, this invention relates to the monoclonal antibody NNY128 reacting with human intestinal cancer cells and epitheliocytes of the alimentary canal and with the Tn antigen which is expressed on more than 80 % of epithelial cancer cells and especially reacts ; with a glycopeptide having the following structure :

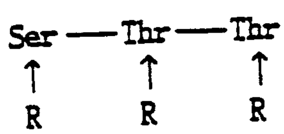

wherein R is GalNAc

It also relates to a hybridoma which stably grows in the abdominal cavity of rats and secretes the monoclonal antibody NNY128.

The monoclonal antibody of this invention was obtained by selecting a hybridoma which produces a monoclonal antibody reacting with mucin type glycopeptides prepared from LS180 cell (ATCC CL180) from hybridomas which are produced by fusing spleen cells of immunized mice with myeloma cells.

Furthermore, this invention provides a glycopeptide which is recognized by the above antibody and has the following structure:

$$\begin{array}{ccccccccccccccccc} Y & - & Leu & - & Ser & - & Glu & - & Ser & - & Thr & - & Thr & - & Gln & - & Leu & - & Pro \\ & & & & \uparrow & & & & \uparrow & & \uparrow & & \uparrow & & & & & & \\ & & & & R & & & & R & & R & & R & & & & & & \end{array}$$

wherein Y is Ala or hydrogen and R is GalNAc.

This glycopeptide is useful for the selection and preparation of a monoclonal antibody such as the above-described antibody recognizing the Tn antigen.

It is generally known that the Tn antigen is expressed on more than 80 % of epithelial cancer cells which are observed in most cancer types (about 90 %). Therefore, the antibody of the present invention is useful for cancer diagnosis such as histochemical diagnosis and in vivo imaging diagnosis. Furthermore, it can be used for cancer therapy by attaching an appropriate agent to it. By using the glycopeptide of the present invention, antibodies recognizing the Tn antigen can be selected efficiently.

Figure 1 shows that NNY128 has an affinity for several kinds of peptides.

The first characteristic of the present invention is that the monoclonal antibody reacts with sugar chains of glycoproteins, especially mucin type glycoproteins. Such antibodies seemed difficult to obtain. The second characteristic is that the antibody belongs to the IgG class reacting with protein A. The advantage of IgG over IgM is that an immunoaffinity chromatography can be performed by IgG and that fragments of F-$(ab')_2$ which are suitable for the in vivo diagnosis of cancer can be prepared from IgG. The immuno affinity chromatography (a means for capturing an antigen with the aid of a column containing antibodies) is the most effective means for determining the epitope.

The antibody of the present invention reacts with tissues of human intestinal cancer and the surrounding parts, but does not react with completely normal tissues. Furthermore, Tn erythrocytes having Tn antigens are strongly agglutinated by the antibody. Furthermore, this antibody is not reactive with any glycolipid extracted from SW1116 (ATCC CCL 233), LS180 (ATCC CL-180) and from cow and pig brains.

The antibody of the present invention is distinct from the other antibodies reacting with mucin such as MLS102 (A. Kurosaka et al., FEBS Letters 215, 137 (1987), J. Biol. Chem. 263, 8724 (1988)), NCC-LU-81 (S. Hirohashi et al., Proc. Natl. Acad. Sci. U.S.A. 82, 7039 (1985)), and CU-1 (H. K. Takahashi et al., Cancer Res. 48, 4361 (1988)).

The antibody of the present invention reacts with LS180 (ATCC CL-180), sheep submaxillary mucin (OSM), and human erythrocytic glycophorin A treated with sialidase and β-galactosidase (from bovine testicles).

Further, the antibody of the present invention has an affinity for each peptide as shown in the examples and Figure 1, and therefore, it is concluded that the antibody recognizes the glycopeptide having the structure represented by the following formula:

$$\begin{array}{ccccc} Ser & — & Thr & — & Thr \\ \uparrow & & \uparrow & & \uparrow \\ R & & R & & R \end{array}$$

wherein R is GalNAc.

and preferably represented by the following formula:

$$\begin{array}{ccccccc} A & — & Ser & — & Thr & — & Thr \\ & & \uparrow & & \uparrow & & \uparrow \\ & & R & & R & & R \end{array}$$

wherein A is an amino acid residue and R is GalNAc. Examples of the "A" of the formula are Glu, Ser, and Leu. More particularly, the antibody of the present invention recognizes the glycopeptide having the structure represented by the following formula:

$$\begin{array}{ccccccc} A\text{ --- } & Ser & \text{ --- } & Thr & \text{ --- } & Thr & \text{ --- } B \\ & \uparrow & & \uparrow & & \uparrow & \\ & R & & R & & R & \end{array}$$

wherein A is an amino acid residue, B is an amino acid sequence, and R is GalNAc.

Specifically, it recognizes the glycopeptide having the structure represented by the following formula:

$$\begin{array}{ccccccc} A\text{ --- } & Ser & \text{ --- } & Thr & \text{ --- } & Thr & \text{ --- } B \\ & \uparrow & & \uparrow & & \uparrow & \\ & R & & R & & R & \end{array}$$

wherein A is Glu, Ser or Leu; B is Gln-Leu-Pro, Gly-Val-Ala or Glu-Val-Ala, and R is GalNAc. However, A and B described in the above formula are not limited to the above.

The hybridoma which secretes the antibody was obtained by fusing spleen cells of mice which had been intraperitoneally immunized with human intestinal cancer cells of LS180 with myeloma cells, followed by selecting a clone which produces an antibody having an affinity for mucin type glycopeptide sugar chains previously prepared from LS180 cells.

It is possible to stably store the hybridomas for a long period of time in a mixture of 10 % dimethyl sulfoxide and 90 % fetal calf serum (FCS) under liquid nitrogen (-198 °C). On the occasion of the use, a portion of the hybridomas is thawed and proliferated in RPMI-1640 or a Dulbecco's minimum essential medium including 20 % FCS. The culture is intraperitoneally implanted in the mice to which pristane had previously been intraperitoneally administered at a ratio of about 0.5 ml per mouse for 2 weeks to 3 months.This way a large quantity of monoclonal antibody NNY128 can be obtained from the ascitic fluid of the mice 10 days to 2 weeks after implantation. In this case, it is possible to stably obtain 7 - 14 mg of the antibody from about 5 ml of the ascitic fluid per mouse. The antibody in the ascitic fluid can easily be purified by fractionation with ammonium sulfate, dialysis, an affinity column chromatography using protein A - sepharose 4B and so on. Further, the monoclonal antibody NNY128 can be obtained by in vitro culture of the hybridoma at a concentration of 20 - 40 µg/ml after about 3 days culturing and, accordingly, the antibody in the in vitro culture is also available. The hybridoma of the present invention was designated as Hybridoma NNY128 and deposited with the Fermentation Research Institute, Agency for Industrial Science and Technology, 1-3, Higashi 1 chome, Tsukuba-shi, Ibaraki-ken, Japan on March 9, 1989 under Accession number FERM BP-2330 in accordance with the Budapest Treaty.

Further, the present invention relates to the glycopeptides GP-2 and GP-3 having the following structure, which are recognized by the present antibody, as shown in the Examples and Figure 1.

$$\begin{array}{cccccccccc} Y- & Leu- & Ser- & Glu- & Ser- & Thr- & Thr- & Gln- & Leu- & Pro \\ & & \uparrow & & \uparrow & \uparrow & \uparrow & & & \\ & & R & & R & R & R & & & \end{array}$$

wherein Y is Ala or hydrogen and R is GalNAc.

The glycopeptide of the present invention is useful for the selection and preparation of monoclonal antibodies recognizing the Tn antigen, such as the above antibody. Particularly, if the glycopeptide is immobilized on a plate etc. in accordance with the method described in JP Patent Unexamd. Publn. No. 61-212568 as mentioned above, antibodies recognizing the Tn antigen can efficiently be selected.

## Examples

In the following, the present invention will be described in detail by means of specific examples. However, these examples are merely illustrative and do by no means limit the scope of the present invention.

## Example 1

### Preparation of Hybridoma

LS180 cells were used as an immunogen for immunizing Balb/c mice.

LS180 cells cultivated in Eagle's medium containing 10 % fetal calf serum were intraperitoneally implanted 9 times to Balb/c mice within 3 months (1-2 x $10^6$ cells in 0.5 ml of physiological saline per mouse per implantation). Next, 1-2 x $10^6$ cells suspended in 0.5 ml of physiological saline were intravenously implanted in each mouse. The spleen cells were obtained from the mice 3 days after the last implantation. A mixture of 1.5 x $10^8$ spleen cells and 2 x $10^7$ myeloma cells (SP2/0-Agl4) in Dulbecco's medium not containing fetal calf serum was centrifuged (1000 rpm, 5 min.) for washing, mixed with 0.5 ml of Dulbecco's medium containing 35 % of polyethylene glycole (PEG, #1000) but not fetal calf serum, and then centrifuged (1000 rpm, about 5 min.) to fuse the spleen cells and the myeloma cells. The PEG used for the fusion was diluted with 5 ml of Dulbecco's medium not containing fetal calf serum and then with 5 ml of Dulbecco's medium containing 20 % fetal calf serum and thereafter removed by centrifugation (1000 rpm, 5 mins.). The cells without PEG were suspended in 50 ml of a mixture of 72 % Dulbecco's medium and 8 % NCTC109 medium (HT medium), a drop (about 0.1 ml) of which was placed into each well of 96 well-tissue culture plates and cultivated for 24 hrs. Then a drop of HT medium containing 0.8 $\mu$M of aminopterin was placed into each well to give HAT medium. The hybridomas obtained by the fusion were selected from the spleen cells and the myeloma cells which did not fuse by cultivation in the HAT medium. About 10 days after cell fusion, a supernatant of the culture of each well was picked up and assayed for its affinity to G-50I (see Referential Example) adsorbed on wells by the method to be described in Example 4. In the case of the wells containing clones which reacted with G-50I , the cloning by the dilution method was repeated twice, whereby the clone producing the antibody named NNY128, which strongly reacts with G-50I but not at all with G-50II or glycolipid and, further, reacts with G-50I without sialic acid, was selected.

## Example 2

### Preparation and Purification of Monoclonal Antibody

To Balb/c mice 0.5 ml of pristane per mouse was intraperitoneally administered. About 1 month later, $10^7$ cells of the hybridoma secreting NNY128, which had been cultivated in a hybridoma proliferation medium consisting of 10 % Dulbecco's medium, 10 % NCTC109 medium, and 20 % fetal calf serum, suspended in 0.5 ml of Hank's biological saline (8g NaCl, 0.4g KCl, 0.14g $CaCl_2$, 0.1g $MgSO_4 \cdot 7H_2O$, 0.1 g $MgCl_2 \cdot 6H_2O$, 0.06g $KH_2PO_4$, 0.06g $Na_2HPO_4 \cdot 12H_2O$, 1g Glucose, and 0.35g $NaHCO_3$ per liter) were intraperitoneally implanted in each mouse. The ascitic fluid was obtained from the mice about 10 days after implantation. In this case, about 10 mg of NNY128 was contained in about 5 - 7 ml of the ascitic fluid per mouse. To the ascitic fluid ammonium sulfate was added to give 50 % saturated solution. A precipitate obtained by centrifugation of the mixture was dissolved in 0.1 M borate Buffer (pH 8.2, Buffer A) and dialyzed against Buffer A. Next, the resultant was divided into several groups and each group was loaded on a column of protein A - Sepharose CL-4B and washed with Buffer A. The column had previously been equilibrated by Buffer A and contained 6 ml of resin capable of adsorbing about 30 mg of IgG. The antibody adsorbed on the column was eluted with 0.1 M acetic acid containing 0.15 M sodium chloride. The eluate was neutralized with 1 M Tris and ammonium sulfate was added until 50 % saturation, and the mixture was centrifuged to give a precipitate. The precipitate was dissolved in buffer A and dialyzed against Buffer A to give the purified monoclonal antibody NNY128.

## Example 3

### Affinity of Monoclonal Antibody NNY128 for Human Intestinal Cancer Tissue

Human intestinal cancer tissue fixed with formalin was dehydrated and covered with paraffin. A thin slice was cut off from said tissue and the paraffin was removed from the thin slice with xylene. The slice was then washed with alcohol to remove the xylene, washed with phosphate bufferred saline (PBS) and then soaked in PBS. The slice was allowed to react with the monoclonal antibody NNY128 overnight. After washing, the slice was allowed to react with anti-mouse IgG antibody labeled with Fluorescein isothiocyanate (FITC). After removing the unreacted FITC-IgG, the part which reacted with NNY128 was examined by means of the fluorescence microscope. NNY128 was found to react with cancer tissues and the surrounding parts but not with completely normal tissues.

## Example 4

Affinity of Monoclonal Antibody NNY128 for Mucin Type Glycopeptide

Mucin type glycopeptides (G-50I) isolated from human intestinal cancer cells (LS180) were immobilized on wells of poly(vinyl chloride) through polylysine and glutaraldehyde. 1 % bovine serum albumin was added to the wells and allowed to stand for 1 hr. After washing the wells, monoclonal antibody NNY128 was added to the wells and allowed to react overnight. After washing the wells, protein A labeled with $^{125}I$ was added to the wells and allowed to react for 2 hrs. After washing the wells, an affinity of NNY128 was measured by using radioactivity of $^{125}I$ bound to the wells as an index. NNY128 was formed to react with G-50 I.

Even when G-50I without sialic acid was used in the same way, NNY128 showed similar affinity to it. This proved that NNY128 reacts with the sugar chain of mucin type glycopeptide not containing sialic acid.

It was also possible to measure an affinity of NNY128 by the method using polystyrene beads. Polystyrene beads (EP-03, #80, SEKISUI) were washed with phosphate buffered saline (pH 7.4, PBS). The beads were allowed to stand overnight at 4 °C in PBS containing 0.2 mg/ml of NNY128 to coat the beads with the antibody. The beads were washed 3 times with PBS containing 5 % bovine serum albumin (BSA) and soaked in the same solution for 1 hr. After removing the solution, 100 µl of test solution (containing LS180 G-50I), 50 µl of PBS solution containing 0.2 % Tween 20, and 50 µl of PBS containing 12.5 ng of $^{125}I$ - NNY128 (about 200,000 counts) and 0.1 % BSA were well mixed with the beads and the mixture was allowed to stand overnight at 4 °C. Then the beads were washed 3 times with PBS and the radioactivity values of the beads were measured by a $\gamma$-counter.

## Example 5

Affinity of Monoclonal Antibody NNY128 for OSM

Asialo OSM obtained by treating OSM with sialidase reacted with NNY128, while OSM treated with $\alpha$-N-acetyl galactosaminidase lost the activity. Therefore, the following experiment was performed in order to isolate the epitope containing GalNAc from OSM.

As the reactivity of OSM was extremely decreased by the treatment with pronase, trypsin having a narrow specificity was used to isolate the epitope. First, asialo OSM was prepared from OSM by digestion with trypsin. Twenty mg of asialo OSM in 20 ml of buffer (50 mM tris hydrochloric acid and 20 mM calcium chloride, pH 8) were mixed with 600 µg of trypsin and incubated at 37 °C for 24 hrs. The mixture was loaded on a column of NNY128 - protein A - sepharose 4B (prepared by combining 6 ml of protein A - sepharose 4B (Pharmacia) with 60 mg of NNY128) and the column was thoroughly washed with 30 ml of 50 mM phosphate buffer (pH 7.2) and the adsorbate was eluted with 30 ml of 50 mM diethylamine (pH 11.5). After freeze-drying the eluate, it was dissolved in a small amount of 0.1 % trifluoroacetic acid and chromatographed on a column of HPLC (Waters, µbondapak $C_{18}$). By monitoring the absorption of UV light at 210 nm a single peak (GP-1) at a location of about 27 min. of elution time was obtained. Using about 1 nmol of the eluate, an amino acid sequence was determined with a Gas Phase Protein Sequencer (Applied Science Co., 477A). The result is shown in Figure 1. The sequence of 51 residues was determined but X positions were not detected as amino acid because of combination with sugars. As a reference, an amino acid sequence of peptide T1 of apo OSM (prepared by chemically completely removing the sugar chains from OSM) was used (R. L . Hill et al., J. Biol. Chem. 252, 3799-3804, (1977)). Both amino acid sequences except for the X positions coincided with each other completely. Therefore, the amino acid residues at the X positions were identified as serine or threonine. GP-1 is too large to be an antigen. Therefore, GP-1 must be fragmented further to obtain suitable epitopes.

As GP-1 contains a glutamic acid residue, GP-1 was digested with V8 protease (Boehringer Mannheim) which exhibits a specificity to a glutamic acid residue.

HPLC confirmed that GP-1 was divided into two fragments, that is, GP-1A and GP-1B. When the fragments were subjected to an affinity chromatography using a column of NNY128 - protein A -sepharose 4B, GP-1A passed through the column and GP-1B was eluted a little later than GP-1A because of the weak affinity for the antibody. Amino acid sequences of GP-1A and GP-1B are shown in Figure 1. As is apparent from the Figure, GP-1B lost the affinity for the antibody by the removal of an N-terminal glutamic acid residue. Further, asialo OSM was digested by thermolysin in order to maintain a glutamic acid residue followed by a sequence of X-X-X. A solution of 200 μg of asialo OSM dissolved in 200 μl of buffer (20 mM Tris hydrochloric acid, 50 mM sodium chloride, and 2.5 mM potassium chloride, pH 7.6) was mixed with 6 μg of thermolysin (Seikagaku Kogyo Co., Ltd.) and incubated for 24 hrs. The mixture was subjected to an affinity chromatography on a column of NNY128 - protein A - sepharose 4B. After washing sufficiently, the adsorbate was eluted with 30 ml of 50 mM diethylamine (pH 11.5). By HPLC analysis, detected in the eluate, that is, the peaks of GP-2 and GP-3 were found at an elution time of about 17 mins. and about 18 mins., respectively, and each was recovered. Their amino acid sequences are shown in Figure 1. GP-2 consists of nine amino acid residues and GP-3 consists of ten amino acid residues and it was found that they contain the sequence Glu-X-X-X (Glu-Ser-Thr-Thr).Although a GalNAc - binding site exists in the 17th amino acid residue (Ser), the epitope seems to be glycopeptide containing Ser-Thr-Thr bound to GalNAc by a comparison with the structure of Glycophorin A of erythrocytes as described below.

## Example 6

### Affinity of Monoclonal Antibody NNY128 for Glycophorin A

An N-terminal amino acid sequence of glycophorin A is shown in Figure 1. Serine and threonine residues at the positions 2, 3, 4, 10, 11, 12, 13, 14, and 15 are bound to galactoses and sialic acids through GalNAcs bound to these amino acids. Therefore, glycophorin A as such does not react with NNY128 or an antibody which has been called an anti-Tn antibody. Glycophorin A treated as follows, however, shows strong reactivity with NNY128.

A solution of glycophorin A (1 μg/100 μl) ( SIGMA CHEMICAL COMPANY) dissolved in 50 mM phosphate buffer (0.14 M, pH 7.4) was immobilized on a plate of poly(vinyl chloride) (JP Patent Unexamd. Publn. No. 62-212568) and 0.5 mU of sialidase (NACALAI CHEMICAL COMPANY) dissolved in 100 μl of 0.2 M acetate buffer was added to the plate, which was then incubated for 2 hrs, at 37 °C and sufficiently washed with the same buffer. Further, 10 mU of ox or bovine testicles β-galactosidase (Boehringer Mannheim) was added thereto and incubated for 24 hrs at 37 °C, followed by addition of 5 mU of the galactosidase. NNY128 and then $^{125}I$ - protein A were added thereto, whereby a quantity of bound NNY128 was measured. As a result, 1,000 counts were detected in an untreated glycophorin A compared to 50,000 counts in an enzyme-treated glycophorin A.

It is not clear whether GalNAc residues continuously bound to glycophorin A at the locations 10 - 15 are an epitope or not.

## Example 7

### Determination of Epitope

It was confirmed in the above Examples that the antibody of the present invention strongly reacts with GP-1, GP-2, GP-3, and glycophorin A (M type and N type) treated with sialidase and β-galactosidase.

The structure these peptides have in common as shown in Figure 1 is the following:

$$\begin{array}{ccccc} \text{Ser} & - & \text{Thr} & - & \text{Thr} \\ \uparrow & & \uparrow & & \uparrow \\ \text{R} & & \text{R} & & \text{R} \end{array} \qquad \text{Formula I}$$

wherein R is GalNAc.

The antibody of the present invention weakly reacts with GP-1B which has the above structure. It reacts strongly with sialidase- and β-galactosidase-treated glycophorin A (M type and N type) which has the above structure supplemented with an amino acid residue at the N-terminus of the peptide chain. Accordingly, the antibody of the present invention recognizes glycopeptides having the structure represented by the following formula:

$$\begin{array}{ccccccc} \text{A} & - & \text{Ser} & - & \text{Thr} & - & \text{Thr} \\ & & \uparrow & & \uparrow & & \uparrow \\ & & \text{R} & & \text{R} & & \text{R} \end{array} \qquad \text{Formula II}$$

wherein A is an amino acid residue and R is GalNAc. As shown in Figure 1, Glu, Ser, or Leu are exemplified as the "A" in the formula. However, the "A" should not be limited to the above amino acid residues because the antibody of the present invention not only recognizes the structure of Formula II effectively whenever "A" is Glu, Ser, or Leu but also irrespective of the kind of the "A".

Further, the antibody of the present invention weakly reacts with GP-1B having the structure of Formula I without the "A" of Formula II . Therefore, antibodies recognizing glycopeptides with the structure of Formula I fall within a scope of the present invention.

It seems important, but not essential, that the structure recognized by the antibody of the present invention should have an amino acid sequence at the C-terminus of the peptide chain of the above Formula II . That is, the antibody of the present invention recognizes the glycopeptide having the structure represented by the following formula:

$$\begin{array}{ccccccccc} \text{A} & - & \text{Ser} & - & \text{Thr} & - & \text{Thr} & - & \text{B} \\ & & \uparrow & & \uparrow & & \uparrow & & \\ & & \text{R} & & \text{R} & & \text{R} & & \end{array} \qquad \text{Formula III}$$

wherein A is an amino acid residue, B is an amino acid sequence, and R is GalNAc.

As shown in Figure 1, Gln-Leu-Pro, Gly-Val-Ala, and Glu-Val-Ala are examples of the "B" of the above formula III . The "B" should not be limited to the above amino acid sequences, because the structure of Formula III is effectively recognized by the antibody of the present invention irrespective of the kinds of the "B" and the "B" may be any amino acid sequence.

Example 8

Affinity of Monoclonal Antibody NNY128 for Tn Erythrocyte

Freshly collected blood was diluted twice with Alsever solution containing an anti-blood agglutination agent to give blood suitable for storage. This blood contained about 20 % of erythrocytes (packed volume after centrifugation at 2,000 rpm for about 10 min). A suspension of erythrocytes was prepared using the storable blood as an original material. A suspension of 5 % erythrocytes was washed three times with PBS to give a suspension of 2 % erythrocytes. Then, 25 μl of 2 % erythrocytes suspension were added to serially diluted solutions of the antibody (2-fold dilution, 25 μl), which had previously been prepared using a microplate. After shaking and stirring, the mixtures were allowed to stand for 2 hrs at room temperature and examined for agglutination. NNY128 antibody agglutinated Tn erythrocytes but no normal erythrocytes (A, B, and O types). A maximum dilution for positive agglutination was $2^4$-fold ($2^0$ : hybridoma spent medium original solution ). Compared with other anti Tn monoclonal antibodies (i.e. NCC-LU35 (IgM)) and CA3239 (IgM)), the agglutination of Tn erythrocytes by NNY128 was weak.This seems due to a difference between the classes of antibodies, namely a difference between IgG and IgM.

Example 9

Affinity of Monoclonal Antibody NNY128 for Glycolipids Cow and pig brains, SW1116 cells, and LS180 cells were homogenized with a Dounce type homogenizer at 4 °C in water. To the homogenate were added methanol and chloroform until the final concentration of the mixture of chloroform / methanol / water reached 2.7 : 5.4 : 2 (v/v). The mixture was stirred for 30 mins. at room temperature for extraction of glycolipids. After centrifugation, 2 volumes of water were mixed with the obtained precipitate. After homogenization, 8 volumes of a solution of chloroform / methanol (1 : 2 v/v) were added to the mixture and stirred for extraction of glycolipids. The mixture was centrifuged to give an extract. After filtration of the above 2 extracts with Celite 535, the extracts were mixed in a separating funnel and water was added thereto to give a mixture containing chloroform / methanol / water (1 : 2 : 2.4 (v/v)). After stirring the mixture gently, allowing it to stand, and collecting the upper layer, the lower layer was mixed with methanol equivalent to 3 times its water volume, stirred sufficiently, mixed with 0.001 M potassium chloride equivalent to twice the volume of the lower layer, further stirred gently, and then allowed to stand overnight, and the upper layer was collected. The upper layer was mixed with the previously prepared upper layer and isobutanol was added thereto, followed by drying in vacuo. The dried preparation was stirred overnight in a mixture of chloroform / methanol / water (60 : 30 : 4.5 (v/v)). After centrifugation, a supernatant without insoluble materials was dried and dialyzed against a small amount of water. The dialyzate was dried, dissolved in methanol and centrifuged to give a fraction of glycolipids without insoluble materials. To the fraction of glycolipids was added methanol to give 20 µl of methanol solutions containing glycolipid which was included in 1 mg of starting material (wet weight). Further, 20 µl of the methanol solution were poured into wells of poly(vinyl chloride). Methanol was evaporated under reduced pressure, thereby coating the wells with the glycolipids. To the wells 1 % bovine serum albumin was added and incubated for 30 mins. After washing the wells, the monoclonal antibody NNY128 was added to the wells and allowed to react for 3 hrs. After washing again, $^{125}$ I-protein A was added to the wells and allowed to react for 2 hrs. After the wells were washed, the affinity of the antibody was measured by the radioactivity bound to the wells. NNY128 was found not to have reacted with any glycolipid.

Referential Example

Preparation of G-50 I

G-50 I is a glycopeptide prepared from mucin type glycoprotein wich is part of the cell membranes (protoplasmic membrane) of human intestinal cancer cells LS180 (ATCC CL-187) and is secreted at the outside of the cells.

Human intestinal cancer cells LS180 (ATCC CL-187) were cultivated for 7 days in Dulbecco's medium containing 10 % fetal calf serum and then harvested. After being washed repeatedly with phosphate buffered saline (137 mM sodium chloride, 2.7 mM potassium chloride, 8.1 mM disodium hydrogenphosphate, 1.5 mM potassium dihydrogenphosphate, 1.0 mM potassium chloride, and 0.5 mM magnesium chloride, hereinafter abbreviated as PBS), the cells were suspended in PBS containing 1 % Tritone X-100 (Rohm and Haas Company) and stirred under ice-cooling. After centrifugation, the supernatant was dialyzed and freeze-dried. The resultant was defatted with chloroform / methanol (2 : 1 (v/v)), suspended in acetate buffer containing 0.01 M calcium acetate, and mixed with pronase P (Kaken Pharmaceutical Co., Ltd.) equivalent to one fiftyth the volume of the freeze-dried material. After adding a small amount of toluene, the mixture was allowed to stand at 37°C for 3 days for sufficient proteolysis. To the resultant almost transparent soluble cell digest, the same volume of 10 % trichloroacetic acid was added and the mixture was stirred and centrifuged to remove insoluble materials formed on being allowed to stand. To the supernatant the same volume of ether was added and the mixture was stirred sufficiently. After centrifugation, the supernatant was removed. Then, ether was added to the water layer for extraction of trichloroacetic acid, followed by centrifugation. This procedure was repeated three times. After the water layer reached pH 5, the water layer was loaded on a column (1.3 x 60 cm) of Sephadex G-25, which had previously been equilibrated with 0.5 M pyridine - acetate buffer (pH 5.0). The column was eluted with the same buffer to collect an effluent in a fraction collector. Fractions which were positive in the orcinol-sulfuric acid reaction were combined and freezed-dried. The obtained dried powder was dissolved in 0.5 M pyridine - acetate buffer (pH 5.0), gel-filtered with sephadex G-50 as described above, and divided into 5 ml-fractions using a fraction collector. The fractions (G-50I) passing through the column mainly consisted of glycopeptide derived from mucin type (O-glycoside type) glycoprotein. The fractions Nos. 20 - 29 were combined and freeze-dried to remove the solvent to give mucin type glycopeptide G-50I. The fractions Nos. 30 - 56 were glycopeptide fractions derived from serum type (N-glycoside type) glycoprotein. The serum type glycopeptide is named G-50II.

## Claims

1. A monoclonal antibody recognizing a glycopeptide having the structure represented by the following formula I :

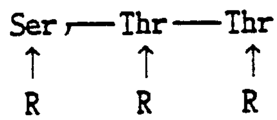

Formula I

wherein R is GalNAc.

2. A monoclonal antibody of Claim 1 recognizing a glycopeptide having the structure represented by the following formula II :

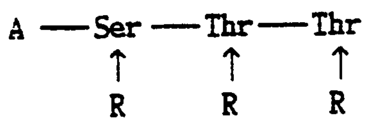

Formula II

wherein A is an amino acid residue and R is GalNAc.

3. A monoclonal antibody claimed in Claim 2, wherein A of the above formula II is Glu, Ser, or Leu.

4. A monoclonal antibody recognizing a glycopeptide having the structure represented by the following formula III :

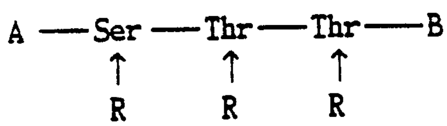

Formula III

wherein A is an amino acid residue, B is an amino acid sequence, and R is GalNAc.

5. A monoclonal antibody claimed in Claim 4, wherein A of the above formula III is Glu, Ser, or Leu; B of that is Gln-Leu-Pro, Gly-Val-Ala, or Glu-Val-Ala.

6. A monoclonal antibody claimed in any of Claims 1 to 5, which belongs to IgG.

7. A monoclonal antibody claimed in any of Claims 1 to 6, which is produced by hybridoma NNY128 (FERM BP-2330).

8. A hybridoma producing the monoclonal antibody claimed in any of Claims 1 to 7.

9. A hybridoma claimed in Claim 8, which is hybridoma NNY128.

10. A glycopeptide represented by the following formula IV :

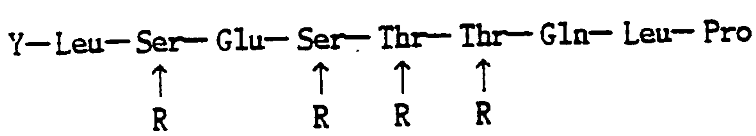

Formula IV

wherein Y is Ala or hydrogen and R is GalNAc.

Claims for the following Contracting State: ES

1. A process for preparing a monoclonal antibody recognizing a glycopeptide having the structure represented by the following formula I:

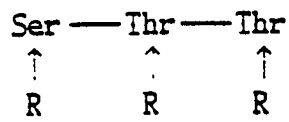

Formula I

wherein R is GalNAc, comprising the following steps: fusing myeloma cells and spleen cells of mice immunized with human intestinal cancer cells, selecting from the resulting hybridoma an anti-mucin type glycopeptide antibody producing hybridoma, cloning said hybridoma by limiting dilution to obtain clones which produce monoclonal antibodies which strongly react with the mucin type glycopeptide, multiplying said clone and recovering said monoclonal antibody.

2. The process according to claim 1 comprising the following steps: implantation of the clone in the abdominal cavity of a mouse previously treated with pristane, where said clone is propagated and produces the monoclonal antibody, collecting the ascites fluid containing the monoclonal antibody and purification of the monoclonal antibody.

3. The process according to claim 1 or 2 whereby the monoclonal antibody obtained recognizes a glycopeptide having the structure represented by the following formula II:

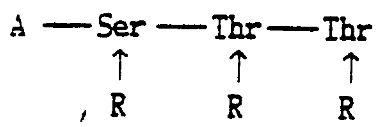

Formula II

wherein A is an amino acid residue and R is GalNAc.

4. The process according to claim 3, wherein in the glycopeptide recognized by the monoclonal antibody A is Glu, Ser, or Leu.

5. The process according to claim 1 or 2, wherein the monoclonal antibody obtained recognizes a glycopeptide having the structure represented by the following formula III:

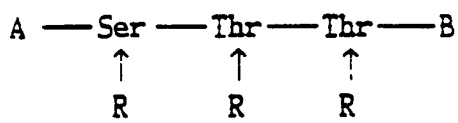

Formula III

wherein A is an amino acid residue, B is an amino acid sequence, and R is GalNAc.

6. The process according to claim 5, wherein in the glycopeptide recognized by the monoclonal antibody A of the above formula III is Glu, Ser, or Leu; B of that is Glm-Leu-Pro, Gly-Val-Ala, or Glu-Val-Ala.

7. The process according to any of claims 1 to 6, wherein the monoclonal antibody obtained belongs to the IgG class.

8. The process according to any of claims 1 to 7, wherein the monoclonal antibody is produced by hybridoma NNY128(FERM BP-2330).

9. Process for producing a hybridoma producing a monoclonal antibody according to any of claims 1 to 8, comprising the steps of fusing myeloma cells and spleen cells of mice immunized with human intestinal cancer cells and selecting from the resulting hybridoma an anti-mucin type glycopeptide antibody producing hybridoma.

10. The process claimed in claim 9, for producing hybridoma NNY128 (FERM BP-2330).

11. A process for preparing a glycopeptide represented by the following formula IV:

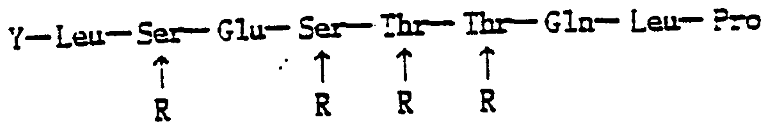

Formula IV

wherein Y is Ala or hydrogen and R is GalNAc, which comprises treating asialo sheep submaxillary mucin (OSM) with thermolysin, subjecting the resulting product to an afinity chromatography on a column containing the monoclonal antibody obtained according to any of claims 1 to 8, and recovering the glycopeptide.

## Figure 1

| | 1 2 3 4 | 14 15 16 17 18 19 20 21 22 23 24 25 26 | affinity |
|---|---|---|---|
| T1* | $NH_2$-Ser-Ser-Val-Pro··· | ···Gly-Ala-Leu-Ser-Glu-Ser-Thr-Thr-Gln-Leu-Pro-Gly-Val··· | |
| GP-1 | X - X -Val-Pro··· | ···Gly-Ala-Leu- X -Glu- X - X - X -Gln-Leu-Pro-Gly-Val··· | strong |
| GP-1A | X - X -Val-Pro··· | ···Gly-Ala-Leu- X -Glu | none |
| GP-1B | | X - X - X -Gln-Leu-Pro-Gly-Val··· | weak |
| GP-2 | | Leu- X -Glu- X - X - X -Gln-Leu-Pro | strong |
| GP-3 | | Ala-Leu- X -Glu- X - X - X -Gln-Leu-Pro | strong |

```
                              1    2    3    4    5    6    7
                   (M type)  Ser                 Gly
glycophorin A                    -Ser-Thr-Thr-       -Val-Ala        strong
  ( Tn type )      (N type)  Leu   ↑    ↑    ↑   Glu
                                   R    R    R
                                R : GalNAc
```

*: R. L. Hill et al., (J. Biol. Chem. 252, 3799-3804, 1977)

X: Not detected as amino acid because of binding of sugars.

European Patent Office

# EUROPEAN SEARCH REPORT

Application Number

EP 90 10 5923

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 255 367 (I. YAMASHINA)<br>* Page 1, line 34 - page 2, line 38 * | 1-10 | C 12 P 21/08<br>C 12 N 5/12 |
| Y | EP-A-0 268 279 (ONCOGEN)<br>* Page 3, line 4 - page 4, line 40 * | 1-10 | |
| Y | WO-A-8 805 054 (IMPERIAL CANCER RESEARCH TECHNOLOGY LTD)<br>* Page 1, line 1 - page 14, line 14 * | 1-10 | |
| A | EP-A-0 212 403 (SLOAN-KETTERING INSTITUTE FOR CANCER RESEARCH) | | |

TECHNICAL FIELDS SEARCHED (Int. Cl.5)

C 12 P

The present search report has been drawn up for all claims

| Place of search | Date of completion of the search | Examiner |
|---|---|---|
| THE HAGUE | 03-07-1990 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)